# EUROPEAN PATENT APPLICATION

(11) **EP 4 817 603 A1**
(43) Date of publication of application: **07.10.2026**
(21) Application number: 24896718.4
(22) Date of filing: 29.11.2024
(51) Int. Cl.: A61K 31/045, A61K 9/06, A61K 9/08, A61K 9/00, A61P 17/00

(54) **USE OF SOLANESOL IN PREPARATION OF DRUG FOR PREVENTING AND/OR TREATING DERMATITIS**

(30) Priority: 01.12.2023 CN 202311634364
(71) Applicant: Beijing Life Science Academy Co., Ltd., Beijing 102209 (CN); Peking University, Beijing 100871 (CN)
(72) Inventor: CHU, Ming, Beijing 100191 (CN); MA, Yinchao, Beijing 102209 (CN); WANG, Ziyuan, Beijing 100191 (CN); HU, Qingyuan, Beijing 102209 (CN); HOU, Hongwei, Beijing 102209 (CN); CHEN, Huan, Beijing 102209 (CN); FU, Yaning, Beijing 102209 (CN); TIAN, Yushan, Beijing 102209 (CN)
(74) Representative: Winter, Brandl - Partnerschaft mbB
(86) International application number: PCT/CN2024/135650
(87) International publication number: WO 2025/113636

(57) **Abstract**

The present invention relates to the use of solanesol in the preparation of a drug for preventing and/or treating dermatitis. The drug of the present invention may be an oral preparation or a topical preparation, and the effect of the drug is equivalent to the effect of tacrolimus ointment.

## Description

### TECHNICAL FIELD

The present disclosure relates to the technology field of medicine, and particularly relates to the use of solanesol in the preparing of a drug for preventing and/or treating dermatitis.

### BACKGROUND

Dermatitis refers to inflammation of the skin. It is a general term for skin inflammatory diseases. It is not an independent disease and is relatively common clinically. The cause of the disease is complex and may be related to contact with allergens, genetics, immunity, environmental factors, abnormal skin barrier function, etc. The clinical manifestations of this disease are diverse. Patients may experience redness, swelling, dryness or itching of the skin, or blistering, exudation, crusting or scaling of the skin. It mainly includes contact dermatitis, atopic dermatitis, seborrheic dermatitis, neurodermatitis and insect bite dermatitis. At present, there are many types of drugs used in clinical treatment of dermatitis, and different treatment methods are used for dermatitis patients with different symptoms. For patients with localized, mild to moderate dermatitis, topical medications are recommended first. Common topical medications include topical corticosteroids (TCS), topical calcineurin inhibitors (TCI), phosphodiesterase 4 (PDE-4) inhibitors, etc. Short-term treatment with TCS can effectively control symptoms for generalized, severe or stubborn skin lesions, but long-term, large-area use of TCS may lead to adverse skin and systemic reactions. TCI and PDE-4 can be used to control inflammation and relieve itching in specific areas such as the neck and wrinkles with good therapeutic effects, but some patients may experience adverse reactions such as an intolerable burning sensation. For patients with systemic attacks and moderate to severe dermatitis, systemic drugs are used for treatment. These mainly include oral antihistamines, immunosuppressants, systemic TCS, biological agents, and Janus kinase (JAK) inhibitors. Although traditional drugs such as TCS and immunosuppressants have shown good effects in treating dermatitis, they have obvious side effects and cannot be used for a long time. Therefore, there is an urgent need to develop innovative drugs for the treatment of dermatitis.

Solanesol is an aliphatic terpene alcohol composed of nine isoprene units, which is mainly found in Solanaceae plants such as tobacco, tomatoes and potatoes. Since chemical synthesis of solanesol is difficult due to its long carbon chain, solanesol is mainly obtained by extraction from Solanaceae plants. Tobacco is the richest plant source of solanesol. Among the various organs of tobacco plants, the leaves have the highest solanesol content, followed by the stems and roots. Currently, solanesol is mainly used to synthesize coenzyme Q10, vitamin K2 and anticancer agent synergist N-solanesoyl-N, N'-bis(3,4-dimethoxybenzyl)ethylenediamine (SDB). In addition, solanesol derivative micelles have also been used to deliver hydrophobic drugs. In recent years, studies have found that solanesol has antibacterial, antioxidant and neuroprotective effects, but the targets and mechanisms of action have not yet been elucidated.

### SUMMARY OF THE INVENTION

In order to overcome the defects of the existing technology, in-depth research found that oral or topical solanesol can effectively treat dermatitis.

Therefore, an object of the present disclosure is to provide a use of solanesol in the preparation of a drug for preventing and/or treating dermatitis.

Preferably, the dermatitis is dermatitis induced by 2,4-Dinitrochlorobenzene (DNCB) sensitization, such as atopic dermatitis or contact dermatitis.

Preferably, the drug is an oral preparation or a topical preparation.

Preferably, the oral preparation is prepared using carboxymethyl cellulose solution as a matrix.

Preferably, the oral preparation is prepared using 0.5% carboxymethyl cellulose solution as a matrix.

Preferably, the concentration of the oral preparation is 0.1 to 100 mg/kg, preferably 1 to 25 mg/kg.

Preferably, the topical preparation is selected from tincture, ointment, paste, aerosol, spray, powder, lotion, rinse, liniment, paint, gel, and patch.

Preferably, the topical preparation is an ointment.

Preferably, the ointment has the following formula: 0.1-10 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

Preferably, the ointment has the following formula: 0.2-5 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

Preferably, the ointment has the following formula: 1 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

The present disclosure relates to the use of solanesol in the preparation of a drug for preventing and/or treating dermatitis, in particular dermatitis induced by DNCB sensitization, such as contact dermatitis or atopic dermatitis. The drug of the present invention may be an oral preparation or a topical preparation, and the effect of the drug is equivalent to the effect of tacrolimus ointment.

The solanesol of the disclosure has wide sources, is safe and effective, has low cost and has a clear mechanism.

### BRIEF DESCRIPTION OF THE DRAWINGS

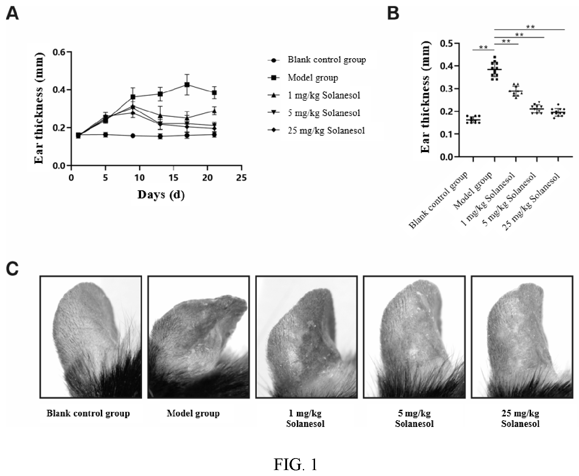

FIG. 1 shows that oral administration of solanesol alleviates ear inflammation in DNCB-sensitized dermatitis mice (**P < 0.01): (A) Dynamic monitoring of ear thickness in dermatitis mice; (B) Measurement of ear thickness in dermatitis mice on day 21; (C) Observation of ear inflammation in dermatitis mice on day 21;

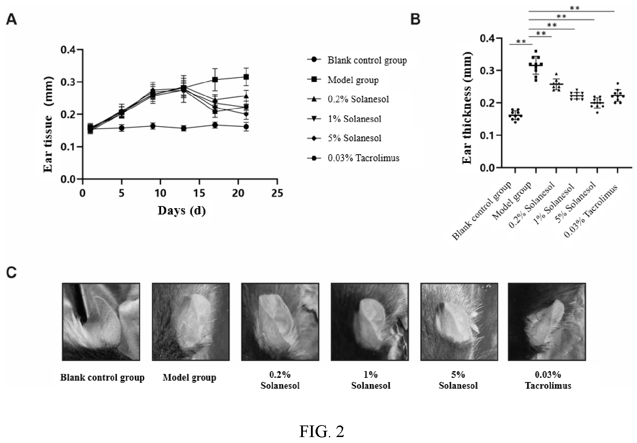

FIG. 2 shows that topical solanesol alleviates ear inflammation in DNCB-sensitized dermatitis mice (**P < 0.01): (A) Dynamic monitoring of ear thickness in dermatitis mice; (B) Measurement of ear thickness in dermatitis mice on day 21; (C) Observation of ear inflammation in dermatitis mice on day 21.

### DETAILED DESCRIPTION OF THE INVENTION

The present disclosure is described below with reference to specific examples. Those skilled in the art could understand that these examples are only used to illustrate the present disclosure, and do not limit the scope of the present disclosure in any way.

### Example 1: Effect of Solanesol in Treating Dermatitis

### 1. Research Plan

### 1.1 Experimental animal

8-week-old SPF-grade C57BL/6J male mice (Animal Ethics Approval No.: LA2022125, Biomedical Ethics Committee of Peking University) were purchased from Charles river (Experimental Animal Production License No.: SCXK(Beijing)2021-0013). Mice were randomly grouped and housed in standardized individually ventilated cages (temperature 25 °C , relative humidity 45-65%, 12-h light/dark cycle) with adequate standardized feed and water.

### 1.2 Establishment of a DNCB-sensitized dermatitis mouse model

Acetone and olive oil were uniformly mixed at a volume ratio of 3:1 to prepare a matrix, and 1% and 0.5% DNCB solutions were prepared based on this matrix. The mice were housed in a clean environment with constant temperature and humidity and were randomly grouped according to the random number table method. Ear dermatitis model in mice modeled by DNCB: in the dermatitis modeling group, 20 µL of 1% DNCB acetone-olive oil solution was applied to the outer skin of both auricles of the mice on the 1st, 3rd, and 5th days of the first week for initial sensitization. Starting from the second week, 20 µL of 0.5% DNCB acetone:olive oil solution was applied to the sensitized area on the outer skin of both auricles of the mice every 3 days for re-sensitization. Drug treatment was performed every day, and sensitization was terminated on the 21st day. The control group was applied with an equal amount of acetone-olive oil matrix at the same time. The thickness of both ears of the mice were photographed and measured using a vernier caliper twice a week at the same time interval.

### 1.3 Grouping and administration

1) Drug Preparation: 0.5% carboxymethyl cellulose solution was used as the matrix to prepare 1, 5, and 25 mg/kg solanesol for oral administration. 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water were dissolved at 75°C and stirred evenly with a magnetic stirrer. This matrix was used to prepare 0.2 w/w%, 1 w/w%, and 5 w/w% solanesol ointment. The ointment was drawn into a 1 mL syringe and slowly cooled to a paste for topical administration. 0.03% tacrolimus ointment was heated until melted and drawn into a 1 mL syringe, and slowly cooled to a paste.
2) Study on oral solanesol treatment of ear model in mice modeled by DNCB: 30 C57BL/6J mice were randomly divided into blank control group, model group, 1 mg/kg solanesol treatment group, 5 mg/kg solanesol-treated group and 25 mg/kg solanesol-treated group (6 mice in each group). Starting from the second week, the control group was gavaged with 0.5% carboxymethyl cellulose solution at the same time every day, and each treatment group was gavaged and administrated according to the weight of mice at the same time every day.
3) Study on topical solanesol treatment of ear model in mice modeled by DNCB: 30 mice were randomly divided into blank control group, model group, 0.2w/w% solanesol treatment group, 1w/w% solanesol treatment group, 5w/w% solanesol treatment group, and 0.03w/w% tacrolimus treatment group (5 mice in each group). Starting from the second week, 12.5 µL of matrix ointment (i.e., matrix ointment without solanesol) was applied to the dorsal side of both ears of the mice in the control group every day. 12.5 µL of solanesol ointment or tacrolimus ointment were applied to the dorsal side of both ears of the mice in the other treatment groups according to their names in the same way.

### 2. Experimental results

### 2.1 Oral administration of solanesol in the treatment of dermatitis mouse model

The results of oral administration of solanesol to treat the dermatitis mouse model are shown in FIG. 1. After DNCB modeling, the ear thickened significantly compared with the blank group (FIG. 1A). Symptoms of ear skin dermatitis were swelling and thickening of the ear, which could be used as an important indicator of the severity of the dermatitis disease. After three weeks of DNCB induction and drug treatment (FIG. 1B, C), the ear thickening was obvious in the dermatitis model group compared with the blank group, with significant statistical differences (P < 0.01). The ear skin showed obvious inflammatory reactions, redness and swelling of ear were obvious, erythema, papules and skin lesions appeared, and scales, blood crusts and pigmentation were visible in the skin lesions. After treatment with various concentrations of solanesol, the ear thickness decreased, with significant statistical differences (P < 0.01). The phenotype of ear skin dermatitis in mice was alleviated, inflammation subsided, and the redness and swelling of the mouse ears were significantly improved, as well as symptoms such as erythema and scales.

### 2.2 Topical application of solanesol in the treatment of dermatitis mouse model

The results of topical application of solanesol to treat the dermatitis mouse model are shown in FIG. 2. After sensitization with DNCB, the ears of each modeling group were significantly thickened (FIG. 2A). After a total of 21 days of DNCB sensitization and drug treatment (FIG. 2B, C), the ears of the model group mice were significantly thickened compared with the blank group mice, with a statistically significant difference (P < 0.01). The ear phenotype showed a significant inflammatory reaction, with obvious dermatitis symptoms such as redness, swelling, and skin lesions, indicating that the disease model was successful. After treatment with various concentrations of solanesol, ear thickness was significantly decreased, with statistically significant differences (P < 0.01). The inflammatory reaction was restrained, and phenotypes such as redness, swelling, and skin lesions were alleviated. There was no statistical difference between low and medium doses of solanesol ointment and 0.03% tacrolimus ointment (P < 0.05), and the phenotype also showed the same therapeutic effect, indicating that topical solanesol has the same therapeutic effect as 0.03% tacrolimus ointment in the treatment of dermatitis. The results showed that local topical solanesol can treat dermatitis, with the optimal dose being 1% solanesol ointment, and it has the same efficacy as known drugs with clear therapeutic effects on dermatitis.

## Claims

1. Use of solanesol in preparation of drug for preventing and/or treating dermatitis.

2. The use according to claim 1, **characterized in that**, the dermatitis is dermatitis induced by 2,4-Dinitrochlorobenzene (DNCB) sensitization, such as atopic dermatitis and contact dermatitis.

3. The use according to claim 1 or 2, **characterized in that**, the drug is an oral preparation or a topical preparation.

4. The use according to claim 3, **characterized in that**, the oral preparation is a preparation prepared by using carboxymethyl cellulose solution as matrix.

5. The use according to claim 4, **characterized in that**, the oral preparation is a preparation prepared by using 0.5% carboxymethyl cellulose solution as matrix.

6. The use according to any one of claims 3-5, **characterized in that**, a concentration of the oral preparation is 0.1-100mg/kg, preferably 1-25 mg/kg.

7. The use according to claim 3, **characterized in that**, the topical preparation is selected from tincture, ointment, paste, aerosol, spray, powder, lotion, rinse, liniment, paint, gel, and patch, preferably, the topical preparation is an ointment.

8. The use according to claim 7, **characterized in that**, the ointment has a following formula: 0.1-10 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

9. The use according to claim 7, **characterized in that**, the ointment has the following formula: 0.2-5 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

10. The use according to claim 7, **characterized in that**, the ointment has the following formula: 1 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

11. A method for preventing and/or treating dermatitis, comprising a step of administering a therapeutically effective amount of solanesol or a drug containing solanesol to a patient in need.

12. The method according to claim 11, **characterized in that**, the dermatitis is dermatitis induced by DNCB sensitization, such as atopic dermatitis and contact dermatitis.

13. The method according to claim 11 or 12, **characterized in that**, the drug containing solanesol is an oral preparation or a topical preparation.

14. The method according to claim 13, **characterized in that**, the oral preparation is a preparation prepared by using carboxymethyl cellulose solution as matrix.

15. The method according to claim 14, **characterized in that**, the oral preparation is a preparation prepared by using 0.5% carboxymethyl cellulose solution as matrix.

16. The method according to any one of claims 13-15, **characterized in that**, a concentration of the oral preparation is 0.1-100mg/kg, preferably 1-25 mg/kg.

17. The method according to claim 13, **characterized in that**, the topical preparation is selected from tincture, ointment, paste, aerosol, spray, powder, lotion, rinse, liniment, paint, gel, and patch,
preferably, the topical preparation is an ointment.

18. The method according to claim 17, **characterized in that**, the ointment has the following formula: 0.1-10 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

19. The method according to claim 17, **characterized in that**, the ointment has the following formula: 0.2-5 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.

20. The method according to claim 17, **characterized in that**, the ointment has the following formula: 1 g of solanesol, 12 mL of olive oil, 3 g of cetyl alcohol, 4 mL of polysorbate 80, 5 g of glyceryl monostearate, 3 mL of silicone oil, 1.5 g of lanolin, 5 mL of glycerol, and 66 mL of water.
